# EUROPEAN PATENT SPECIFICATION

(11) **EP 3 811 991 B1**
(45) Date of publication and mention of the grant of the patent: **25.11.2026**
(21) Application number: 20195148.0
(22) Date of filing: 17.01.2007
(51) Int. Cl.: A61M 5/20, A61M 5/31, A61M 5/32, A61M 5/24

(54) **IMPROVED AUTOINJECTOR SUPPORTING THE SYRINGE AT THE FRONT**
VERBESSERTER AUTOINJEKTOR MIT LAGERUNG DER SPRITZE AM VORDEREN ENDE
AUTO-INJECTEUR AMÉLIORÉ SUPPORTANT LA SERINGUE À L'AVANT

(30) Priority: 23.01.2006 GB 0601309; 07.02.2006 GB 0602411; 21.03.2006 GB 0605644; 22.03.2006 US 387645; 12.10.2006 GB 0620163
(43) Date of publication of application: 28.04.2021
(60) Divisional application: 23173427.8 / 4 218 860
(62) Document number(s) of the earlier application(s) in accordance with Art. 76 EPC: 07704923.7 / 1 998 831
(73) Proprietor: SHL Medical AG, 6302 Zug (CH)
(72) Inventor: STAMP, Kevin, Sheffield, S35 1AR (GB)

(56) References cited:
- WO-A1-00/24441
- WO-A1-2005/115506
- WO-A1-99/22792

## Description

This invention relates to the field of autoinjectors for the administration of liquid medication, for example, interferon.

### BACKGROUND

An autoinjector is an automatic injection device designed to facilitate automated delivery of a dose of medicament to a patient through a hypodermic needle, the injection usually being administered by the patient themselves. An autoinjector works, for example, by delivering an injection automatically upon actuation by the patient pressing a button, moving a lever or part of a housing etc. This is in contrast to a conventional manual syringe where the patient himself needs to directly depress a plunger into a barrel containing medicament in order to effect the injection. The terms "autoinjector" and "injection device" are used interchangeably in the following description.

Examples of autoinjectors are described in WO2003/099358 (Seedlings Life Science Ventures LLC) and WO01/93926 (Mayo Foundation for Medical Education and Research). These are both generally flat devices which are of small size to encourage users to carry the device with them for ready access. GB2396298 (PA Consulting Services Ltd) is an example of a more conventionally-shaped elongate autoinjector, but of relatively complex internal construction.

All three of the above prior art devices have a custom designed medicament chamber therein rather than being built around a standard pre-filled syringe presentation. The custom medicament chamber, although allowing for a . compact overall . size . for. the device, means that the device as whole must be subjected to more rigorous regulatory control as compared with a device containing a standard pre-filled syringe presentation which will have already obtained regulatory approval.

Another reason why the above prior art cannot be used in conjunction with a standard syringe presentation is related to the needle. Usually, a standard pre-filled syringe presentation , to be used within a disposable autoinjector includes a needle in communication with a medicament chamber contained within the barrel of the syringe. It is essential that the sterility and integrity of the needle is maintained and protected right up until the moment it is required to deliver an injection. Usually this is achieved by providing a needle sheath comprising a solid (for example an elastomer such as rubber) sheath into which the needle is staked or spiked so that it is surrounded and sealed on all sides. Usually, at least the forwardmost 3-4mm of the needle is embedded in the rubber of the needle sheath. The autoinjector cannot be operated with the needle sheath in place. Immediately prior to actuating the device, the user removes the needle sheath, for example by removing an endcap from the device to which the needle sheath is attached, so that the needle is ready for use. In contrast, WO01/93926 for example has an entirely different type of needle sheath 79 which is designed to be ruptured by the forward-moving needle during use of the autoinjector. In other words, this type of rupturable needle sheath does not need to be removed from the device before actuation, however, such needle sheaths are likely to provide less mechanical protection than those provided in a standard pre-filled syringe presentation and usually do not directly maintain sterility of the medicament and the needle.

WO 00/24441 (Pharmacia & Upjohn AB) describes an autoinjector for replaceable containers of syringe type and includes a carrier designed to accomodate either of at least two containers of different length and/or width.

WO 2005/115506 (Cilag AG International) describes an injection device having a housing that receives a syringe and includes a return spring for biasing the syringe from an extended position in which its needle extends from the housing to a retracted position in which it does not. A drive spring acts on a first drive element and a second drive element acts upon the syringe to advance it from its retracted position to its extended position and discharge its contents through the needle.

WO 99/22792 (GRECO, Ermanno) describes a self-injection device having a syringe and a disposable needle, and means for preventing the accidental separation of the needle and the consequent expulsion of the needle from the injection device.

In general, an autoinjector includes a needle which is located within the housing of the device. Upon activation of a force-generating source, a portion of the needle extends out of the housing and penetrates the outer layer of skin to deliver medicament. In some known autoinjectors, after activation, a needle cover or needle shield moves forward to conceal the needle after use. In GB2396298, the needle automatically retracts back into the housing by means of a biasing spring. An improved autoinjector is described in our co-pending international patent application, published under number WO 2005/070481. Some of the reference numerals in the present application correspond with the equivalent components in the device described in WO 2005/070481. This device requires that the needle is moved axially so that it can appear beyond the end of the nozzle for the duration of the injection, after which the needle retracts automatically, so that it is never in sight of the user. The device also requires that the plunger is moved axially so that medicament is ejected. The overall complexity of the autoinjector is significantly reduced by both of these requirements being effected by one component, namely an inner housing and the device has the significant advantage that it can be built around a conventional or standard syringe presentation.

The injection device of WO 2005/070481 is designed to be used in conjunction with a standard drug presentation e.g. a pre-filled syringe comprising a needle, barrel pre-filled with medicament and a plunger. The plunger may include a separately-provided plunger rod. As mentioned above, there is a significant commercial advantage in being able to use a standard pre-filled syringe, which will have been subjected to numerous clinical trials, drug stability studies and regulatory approval. Any modification to the standard syringe may require further trials and approval, adding delay and expense. The present invention is relevant to any injection device for use in conjunction with a standard pre- filled syringe presentation (whether preloaded or not and whether single-use or reusable), not only the device described in WO 2005/070481.

The barrel of a syringe is usually glass, since glass has the most favourable storage properties for many drugs. However, glass is notoriously fragile and there is a risk of damage or breakage of the syringe during injection if the forces to which the syringe is subjected by the injection device are not properly controlled. This is particularly so where the liquid medicament is relatively viscous, requiring greater force to expel it from the syringe via the needle. Barrels made of materials other than glass, for example polyethylene or cyclic olefin polymers are less brittle when subjected to normal forces during injection, but still would benefit from the invention described below.

In the known device described in our co-pending patent application no WO 2005/070481 and illustrated in Figures 1-3 of the present application, the syringe is supported within the injection device by a barrel or syringe holder 9. The syringe holder 9 comprises an elongate housing which closely surrounds the glass barrel of the syringe. The annular flange 90 at the rear of the syringe barrel, rests on a barrel seat 91 at the rear of the syringe holder 9. The annular flange 90 at the rear of the syringe barrel is often referred to as a "finger flange" because, during a conventional (manual) injection using a syringe, the user's index and middle fingers rest naturally in front of the "finger flange" in order to provide the necessary resistance to allow depression of the plunger by the thumb to deliver the medicament.

The barrel seat, for example in the form of an annular flange, preferably prevents forward axial movement of the syringe with respect to the syringe holder so that, in use, the syringe barrel and the syringe holder move axially together as one unit.

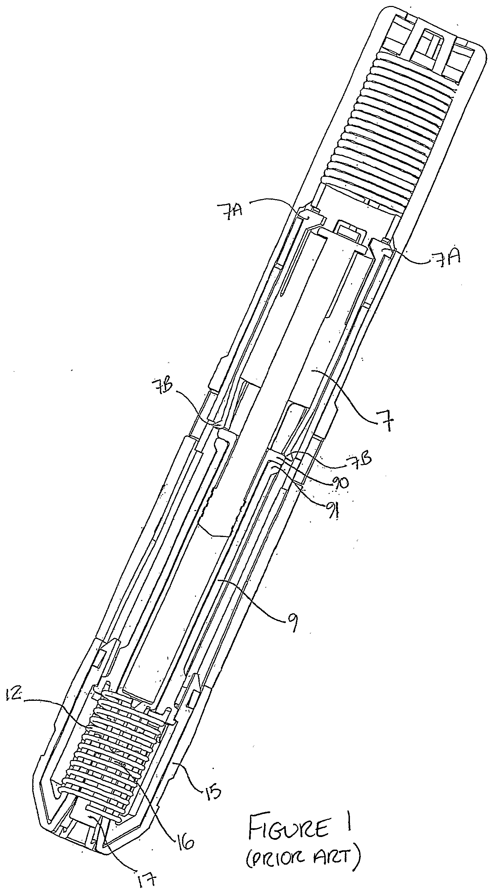

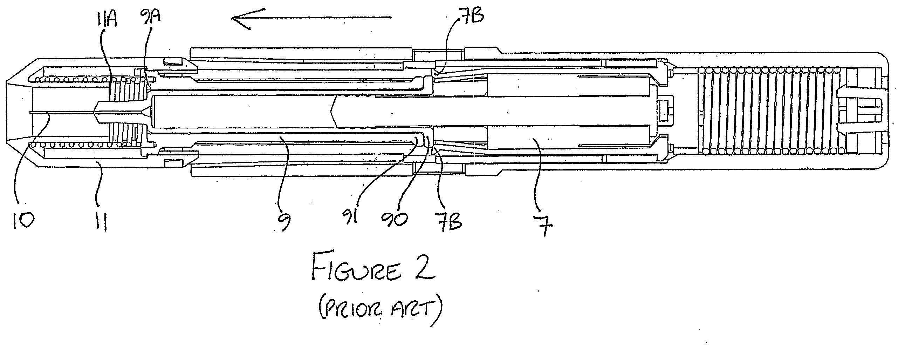

In use, as described in WO 2005/070481, there are three stages of delivering an injection. Before delivering an injection (referring to Figure 1 of the present application), the end cap 15 is pulled off, removing the needle cover 17 (if present) and rubber needle sheath 16 with it from the needle. In the first stage of delivering an injection, as shown in Figure 2 of the present application, the tags 7B at the forward end of the inner housing 7 are in contact with the syringe barrel 90, which is pushed axially forward (taking the syringe holder 9 with it), so that the needle 10, which is fixed to the front end of the barrel, moves in the direction indicated by the arrow so that eventually it protrudes beyond the nozzle 11 at the front of the device. Forward travel of the barrel and syringe holder is limited when a surface 9A of the syringe holder reaches an endstop 11A inside the nozzle or front housing 11.

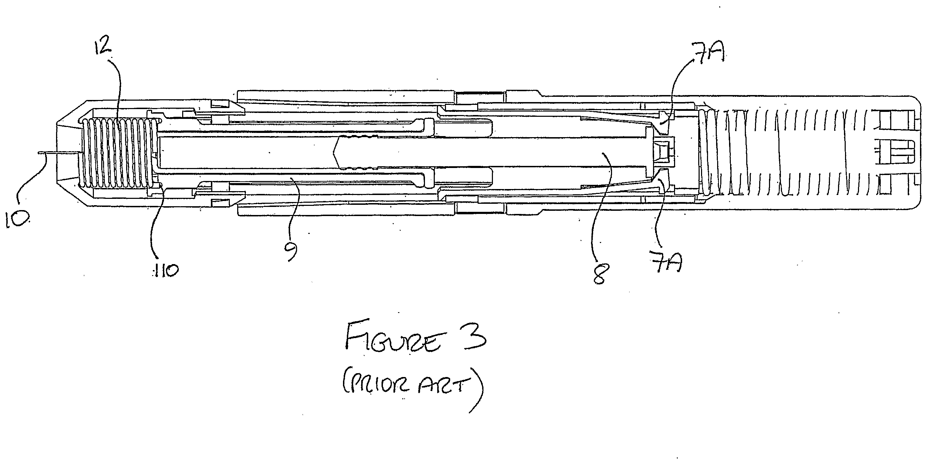

Referring now to Figure 3, the second stage of the injection is the delivery of the medicament wherein the tags 7A at the rear of the inner housing 7 depress the plunger 8 into the barrel of the syringe. During this stage, the barrel of the syringe is held axially stationary, by abutment of the annular "finger" flange 90 against the barrel seat 91, which results in the barrel being placed in tension as the plunger pushes the non- compressible liquid medicament towards the forward end of the barrel. This tension is undesirable in a glass barrel, which may become damaged or broken, especially if the medicament comprises a particularly viscous liquid which requires greater force to expel it from the syringe via the needle. Viscous medicaments are desirable in certain applications, where the use of a sustained-release viscous medicament reduces the frequency that an injection is required.

It is desirable to minimise the diameter of the needle so far as is possible, because the smaller the diameter of the needle, the less painful is the resulting injection. However, for a given length of needle, the smaller the needle diameter, the greater the force required to eject the medicament, from the syringe.

It is also desirable to minimise the duration of the injection, i.e. to maximise the speed at which the medicament is delivered from the syringe. Particularly when the needle diameter is small, minimising the duration of the injection also means an increase in the force used to eject the medicament from the syringe.

An increase in the forces on the syringe consequently increases the likelihood of the syringe breaking during the injection. The risk of the syringe breaking during injection is significant, and is not only inconvenient and costly but is also potentially dangerous. If breakage occurs, it is possible that glass fragments and/or the needle may become detached and exit the front of the device causing injury. Furthermore, there is the risk that the remaining medicament will leak or be ejected from the device in an uncontrolled manner, potentially delivering the wrong dose into the patient, or causing injury e.g. if the medicament contacts the patient's skin or eyes. These problems are amplified when the medicament is viscous as a more powerful energy source is needed in such applications so that the forces involved are greater. It is known that a typical breakage of the syringe during injection would occur at the finger flange, whereby the finger flange 90 on the syringe barrel breaks as a result of its abutment against the barrel seat 91. It is therefore highly desirable to minimise the likelihood of breakage of the syringe.

In the third stage of the injection (not illustrated in the present application but shown in WO 2005/070481), once the medicament has been delivered and the inner housing 7 is no longer in contact with the barrel or plunger of the syringe, the secondary spring 12 pushes the syringe holder (and hence the syringe contained therein) axially rearwardly so as to retract the syringe back into the housing so that the used needle is concealed from view.

### SUMMARY OF THE INVENTION

The invention is defined according to the appended claims.

Preferably, the autoinjector is a single-use autoinjector. The simple construction of the autoinjector makes it very appropriate for applications such as emergency use for injecting a large population to control a pandemic, where a large number of cost- effective disposable autoinjectors are required. A single-use autoinjector also provides a very convenient means for patients to administer their own injections, even if lacking in dexterity and/or clinical experience. Delivery of viscous drugs is particularly problematic for patients lacking dexterity because of the greater. force needed to deliver such drugs - this problem being alleviated by the present invention.

Typically, the autoinjector contains an energy source, for example a coiled spring, for moving said plunger axially in the barrel to deliver an injection in less than 30 seconds.

Preferably, the syringe is axially moveable in said housing and is biased so that the needle is normally wholly inside said housing, wherein before injection the syringe is movable axially so as to move at least a part of said needle out of the housing and wherein after injection, the syringe is able to retract in order to retract said part of said needle into the housing. The concealment of the needle both before and after injection makes the autoinjector particularly suitable where the patient has any aversion to injection by needle. Retraction and retention of the needle after injection eliminates the risk of needle-stick injury.

According to an example, there is provided a method of assembling an autoinjector comprising the steps of:
providing a first part-assembly comprising a front housing and a closely fitting end cap;
providing a second part-assembly comprising a rear part of the autoinjector;
providing a syringe comprising a barrel for holding a volume of medicament, a needle at one end of the barrel and a plunger axially-moveable in the barrel to a forwardmost position;
providing a syringe support means;
inserting the syringe axially into the rear end of the syringe support means until said syringe support means supports the syringe;
inserting the front end of said syringe and syringe support means into said first part-assembly;
assembling said first part-assembly and second part-assembly together so that said syringe support means supports the syringe at an axial location at or forward of the forwardmost position of the plunger, whereby in use said reaction surface provides an axial compressive force on said barrel when a forward axial force is applied to the plunger.

According to a further example, there is provided a method of assembling an autoinjector comprising the steps of:
providing a first part-assembly comprising a syringe support means, a front housing and a closely fitting end cap;
providing a second part-assembly comprising a rear part of the autoinjector;
providing a syringe comprising a barrel for holding a volume of medicament, a needle at one end of the barrel and a plunger axially-moveable in the barrel to a forwardmost position;
inserting the syringe axially into the rear end of the first part-assembly until said syringe support means supports the syringe;
assembling said first part-assembly and second part-assembly together so that, said syringe support means supports the syringe at an axial location at or forward of the forwardmost position of the plunger, whereby in use said reaction surface provides an axial compressive force on said barrel when a forward axial force is applied to the plunger.

### BRIEF DESCRIPTION OF THE DRAWINGS

Preferred embodiments of the present invention will now be more particularly described, by way of example only, with reference to the accompanying drawings in which:
Figure 1 (PRIOR ART) is a perspective view of a known injection device;
Figure 2 (PRIOR ART) is a plan view, partly in section of the Figure 1 device, with the cap and needle cover removed, ready for actuation;
Figure 3 (PRIOR ART) is a plan view, partly in section of the Figure 1 device, with the needle exposed, ready for the plunger to be depressed in order to deliver the medicament;
Figure 4 is a perspective view of an injection device embodying one aspect of the present invention;
Figure 5 is a plan view, partly in section of the Figure 4 device, with the cap, needle cover and needle sheath removed, ready for actuation;
Figure 6 is a plan view, partly in section of the Figure 4 device, with the needle exposed, ready for the plunger to be depressed in order to deliver the medicament;
Figure 7 is a perspective view of the syringe holder;
Figure 8 is a cross-sectional view of the syringe holder of Figure 7;
Figure 9 is a cross-section view of the syringe holder of Figure 7, showing a syringe in place;
Figure 10 is a cross-section view of the syringe holder of Figure 7, showing a syringe, needle sheath and needle cover in place;
Figure 11 shows detail, drawn to a larger scale, of the interface between the front of the glass syringe barrel and the syringe holder illustrated in Figure 10;
Figure 12, drawn to a larger scale, is a cross-sectional view of the modified front housing;

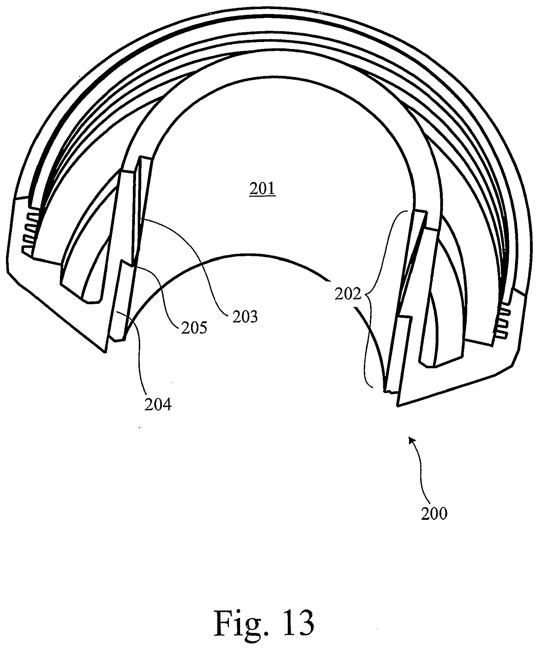

Figure 13, drawn to a larger scale, is a perspective cross-sectional view of the front housing;
Figure 14 is a cross-sectional view of the front housing, cap and syringe holder assembled together;
Figure 15 is a perspective view of the front housing, cap and syringe holder assembled together;
Figure 16 is a cross-sectional view of the front housing, cap, syringe holder and syringe assembled together;
Figure 17 is a cross-sectional view of an alternative embodiment of the syringe holder;
Figure 18 is a part-assembly view showing the syringe holder of Figure 17 together with its spring retainer, a syringe therein and the end cap and front housing;
Figure 19 shows the part-assembly of Figure 18 with the needle cover removed;
Figure 20 is a perspective view of the syringe holder of Figure 17 together with its spring retainer;
Figure 21 is a perspective view of a syringe holder, end cap and front housing, showing an alternative means of retaining the gripping means against the syringe barrel;
Figure 22 is a perspective view of another embodiment of the syringe holder together with a needle cover.

### DETAILED DESCRIPTION

Throughout the description and claims of this specification, the words "comprise" and "contain" and variations of the words, for example "comprising" and "comprises", means "including but not limited to", and is not intended to (and does not) exclude other components, integers or steps.

Throughout the description and claims of this specification, the singular encompasses the plural unless the context otherwise requires. In particular, where the indefinite article is used, the specification is to be understood as contemplating plurality as well as singularity, unless the context requires otherwise.

Throughout the following description, reference to a "forward" direction means the direction which is towards the patient when the injection device is in use. The "forward" end of the injection device is the end nearest the patient's skin when the device is in use. Similarly, reference to a "rearward" direction means the direction which is away from the patient and the "rearward" end of the device is the end furthest from the patient's skin when the injection device is in use.

The "plunger" includes any elastomeric stopper or the like which seals the chamber containing liquid medicament. The plunger typically also includes a plunger rod but this may be provided separately from the elastomeric stopper and need not be an essential part of the syringe. The "forwardmost position" of the plunger refers to the forwardmost position of any part of the plunger (typically the forwardmost edge of the stopper).

Features, integers, characteristics or groups described in conjunction with a particular aspect, embodiment or example of the invention are to be understood to be applicable to any other aspect, embodiment or example described herein unless incompatible therewith.

As described above, a disadvantage of the known prior art is that the barrel of the syringe is placed in tension as the plunger pushes the non-compressible liquid medicament towards the forward end of the barrel for delivery. This tension is undesirable in a glass barrel, which may become damaged or broken, especially if the medicament comprises a particularly viscous liquid which requires greater force to expel it from the syringe. There is a possibility that the glass syringe might break in the region of its finger flanges, as a result of the forces to which it is subjected during delivery of an injection. One way to mitigate this problem is to reduce the effect of those forces in the region of the finger flanges. For example, the syringe holder of WO 2005/070481 can be modified by providing a helical slit at the rear end thereof which, in use, provides resilience to the region of the barrel seat on which is located the finger flange of the syringe (not illustrated). The resilient flexing absorbs shock and reduces the risk of breakage of the syringe in the region of the finger flanges.

Other means for reducing the effect of forces in the region of the finger flanges can be envisaged, for example, providing a cushion in the form of an O-ring or moulding a relatively soft or elastomeric material into a harder substrate in the region of the barrel seat.

It is known that a typical breakage of the syringe during injection would occur at the finger flange, whereby the finger flange 90 on the syringe barrel breaks as a result of its abutment against the barrel seat 91. However, the applicant has recognised that it is also possible that the syringe could break at points on the barrel forward of the finger flange. This is potentially more serious as larger straight glass fragments may be
ejected from the front of the device, as well as the unsecured needle, and any remaining medicament will leak out in an uncontrolled manner.

The risk of breakage or damage to the glass syringe may be reduced by ensuring that the barrel is held in compression during delivery of the medicament (stage two of the injection process described in WO 2005/070481), rather than being in tension. This can be achieved by supporting the forward end of the barrel and having a reaction surface at which an axial compressive force can be applied to the barrel when a forward axial force is applied to the plunger during delivery of the medicament.

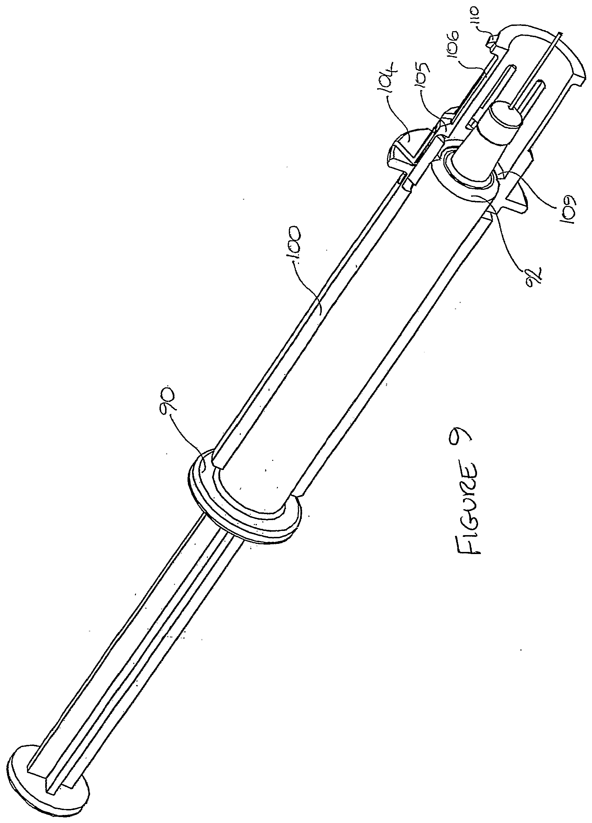

In the injection device of the present invention, the conventional syringe holder 9 is replaced with a more complex syringe holder which is capable of supporting the syringe at the front end of its barrel instead of at its rear flange 90 during delivery of the medicament. In the embodiment described below, the syringe holder supports the syringe at the front shoulder of its barrel and provides a reaction surface there for the front shoulder of the barrel. By "front shoulder" is meant the region at which the largest diameter of the barrel reduces to a smaller diameter at the nozzle. The front shoulder 92 is indicated in Figure 9. Alternatively, the syringe could be supported and the reaction surface provided at the front end of the syringe barrel, or at the narrowed "cone" where the needle is attached thereto and these embodiments are described in more detail below. The syringe can, in fact, be supported at any place on the barrel which, in use, is forward of the forwardmost position of the fully-depressed syringe plunger and where a reaction surface for the syringe can be provided (so that the barrel is held in compression throughout the delivery of the medicament).

However, the desired supporting or holding of the barrel by the syringe holder at its front end presents an assembly difficulty for a product where it is desired to keep manufacturing and assembly costs to a minimum. In the prior art device, the syringe can simply be dropped into the rear end of the syringe holder until its flange 90 rests on the barrel seat 91. This determines the axial position of the syringe with respect to the syringe holder. In practice, the prior art syringe holder is supplied ready-assembled with the front part of the device, so that the pre-filled syringe can simply be dropped into the syringe holder and then the front part of the device (including the syringe) can be attached to the ready-assembled rear part of the device in a simple two-stage assembly operation.

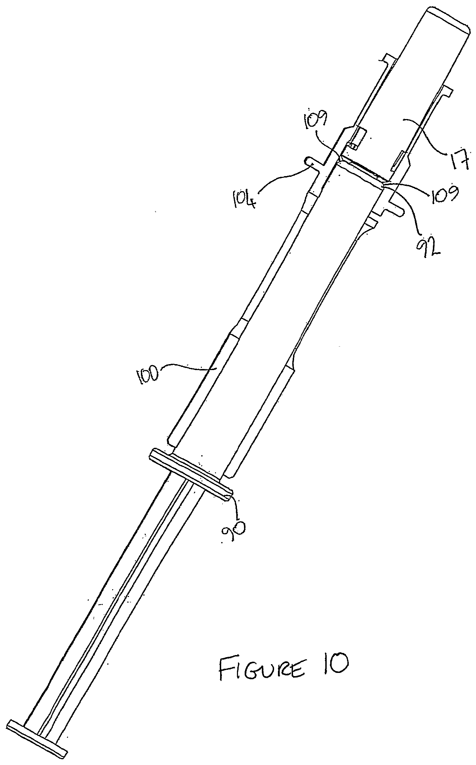

In the present invention, it is not possible to use the finger flange 90 and barrel seat to determine the axial position of the syringe with respect to the syringe holder. This is because the syringe holder needs to actively support the syringe at its front end (preferably at the front shoulder 92 of the glass barrel). In the preferred embodiment there are inwardly-directed gripping means to retain the front shoulder of the glass barrel at a specific axial location with respect to the syringe holder. The standard syringe is usually supplied with a needle cover 17 which typically has a diameter almost the same as the largest diameter of the glass barrel (see Figure 10). The needle cover 17 may be rigid, but is not necessarily so. Inside the needle cover 17, the needle is staked or spiked inside a needle sheath 16, which may be made from rubber. If the syringe is inserted into the syringe holder needle-first (or rather needle-cover first), any inwardly- directed gripping means would foul on the needle cover (or needle sheath if no needle cover is present) as they attempt to pass. There is also the risk of the needle being damaged during assembly, for example if it is pushed into any inwardly-directed gripping means. Such damage to the needle is highly undesirable, as it could cause injury to the patient or could affect or impede delivery of medicament, in particular because it often cannot be readily detected if the needle is concealed within a needle cover and/or a rubber needle sheath. The damage to the needle may only become apparent during delivery of the medicament. The consequent effect of a damaged needle on delivery of medicament may not be apparent to the patient at all.

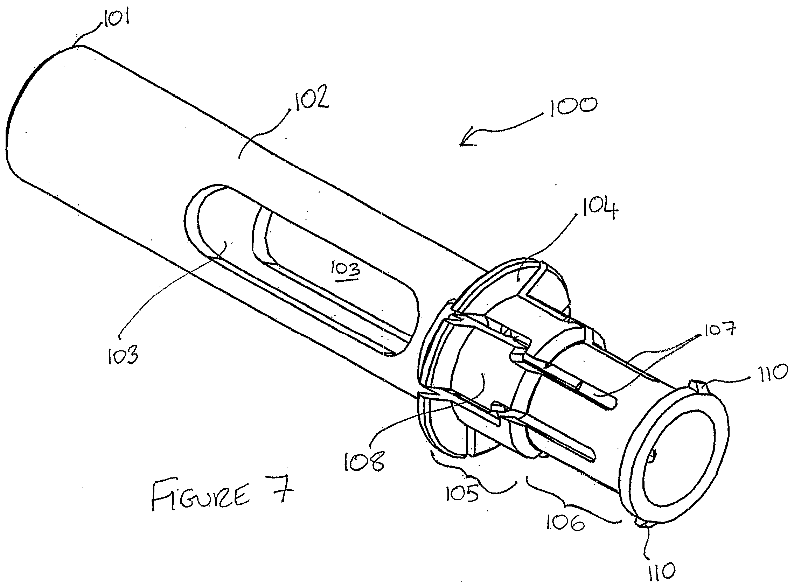

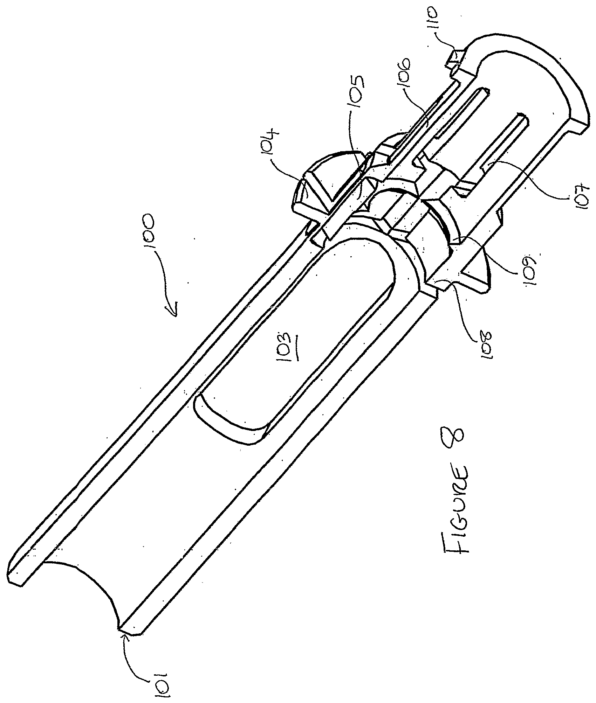

This problem is solved by the use of a syringe holder 100 as illustrated in Figures 7 and 8. The syringe holder 100 comprises an elongate rear portion 102 in which there are viewing windows 103 so that the medicament chamber in the barrel of a syringe held therein would be visible. There is a barrel seat 101 (equivalent to barrel seat 91 in the prior art device) at the rear end of the rear portion 102, but in practice this is not intended to abut the flange 90 of the syringe barrel (unlike in the prior art device).

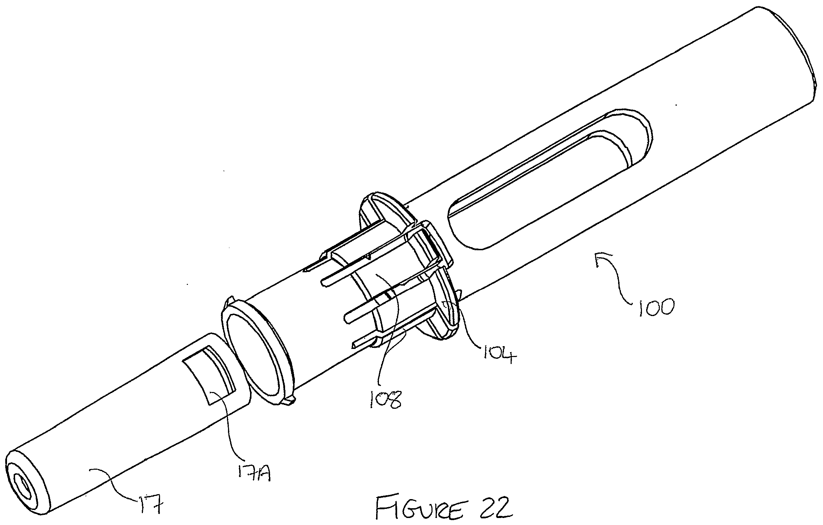

The syringe holder also has an intermediate portion 105 of comparable diameter to the rear portion, and a front portion 106 of narrower diameter. The intermediate portion 105 is provided with a discontinuous annular flange 104. Together, the intermediate and front portions 105, 106 include radially-spaced slots 107 which define a plurality of radially flexible fingers 108. In the illustrated embodiment, there are three flexible fingers 108, but four or some other number of fingers may be provided. Figure 22 shows an embodiment in which four flexible fingers 108 are provided on the syringe holder 100. The four fingers necessarily each are thinner than those in the three-finger embodiment.

A typical .example of a needle cover 17 is shown in Figure 22 (illustrated without the rest of the syringe for simplicity). The needle cover has a slot 17A therein. If the syringe holder 100 is appropriately aligned with the needle cover of a syringe held therein, there is a possibility that one of the relatively thin flexible fingers 108 may undesirably snag or drop into slot 17A, possibly causing damage to the underlying needle that would not become apparent until an injection is delivered. This disadvantage can be overcome by ensuring that each of the flexible fingers 108 is preferably wider than the needle cover slot 17A, as illustrated by the three-finger embodiment of Figure 7.

As shown in Figure 8, gripping means 109 preferably in the form of a discontinuous annular inwardly-directed protrusion, are provided on the interior of the flexible fingers 108 in the intermediate section 105. Referring to Figure 9, when a syringe is located within the syringe holder 100, the gripping means 109 abuts the front shoulder 92 of the barrel in order to define the axial position of the barrel with respect to the syringe holder.

The term "gripping means" is not limited to means which grip radially-inwardly onto the barrel, although in some embodiments they may do so. More important is the fact that the gripping means 109 (or equivalent) supports the syringe in a desired axial location and provides a reaction surface for the syringe so that the barrel will be held in compression during delivery of the medicament.

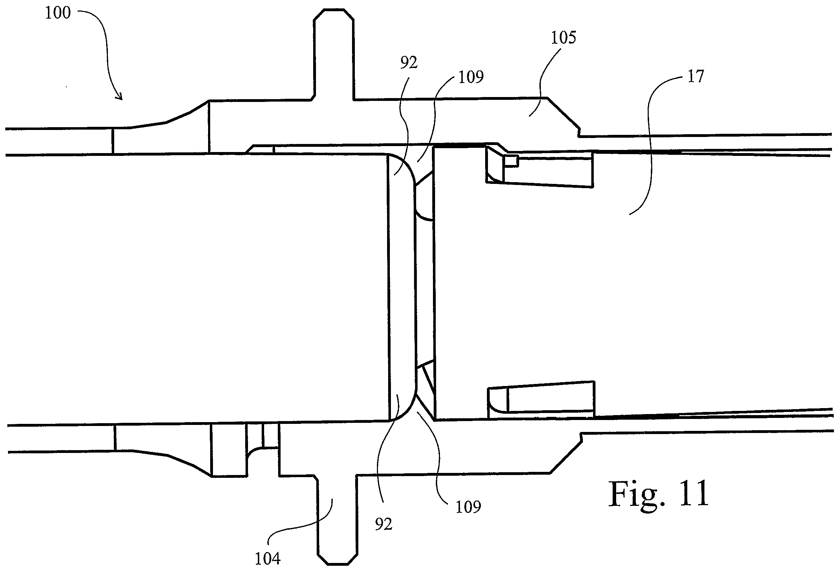

As can be seen from Figures 10 and 11, the needle cover 17 has a greater diameter than the diameter normally available at the gripping means 109. In other words, the internal diameter between the gripping means 109 is smaller than the exterior diameter of the needle cover 17. However, as will be explained in more detail below, when the syringe is inserted into the syringe holder, as the needle cover 17 passes the gripping means 109, the flexible fingers 108 flex radially-outwardly to create sufficient diameter for the needle cover to pass the inwardly-protruding gripping means, without exerting excessive force on the needle therein, thus minimising the risk of damage to the needle. Once the needle cover has passed, the flexible fingers 108 spring back into their normal position (having smaller internal diameter than the exterior diameter of the needle cover) and the gripping means 109 locate at the front shoulder 92 of the barrel to provide the reaction surface against which the syringe will be held in compression during delivery of the medicament. In this position, the gripping means 109 are axially located between the needle cover and the front shoulder of the barrel.

The front portion 106 of the syringe support 100 is provided with a plurality of (preferably two) equispaced tags 110, whose purpose will be described later below.

The most straightforward way to assemble the syringe and injection device is in a three stage procedure, namely:
1. inserting the syringe into the syringe holder, until the gripping means 109 locate at the front shoulder 92 of the barrel;
2. inserting the syringe and syringe holder into the front part of the injection device;
3. assembling the front part to the rear part of the injection device.

Compared with the two-stage assembly procedure of the prior art device, the extra assembly stage is disadvantageous but initially seems necessary as it is not obvious how stages 1 and 2 could be readily combined so that the syringe holder can be supplied ready-assembled with the front part of the device. This is because, once assembled into the front part of the device, the flexible fingers 108 would be prevented from flexing radially outwardly by their necessarily close abutment with the front housing, thus preventing insertion of the syringe and needle cover.

Therefore, in a further embodiment of the invention, a modified front housing for the injection device is provided which enables a two-stage assembly procedure to be used.

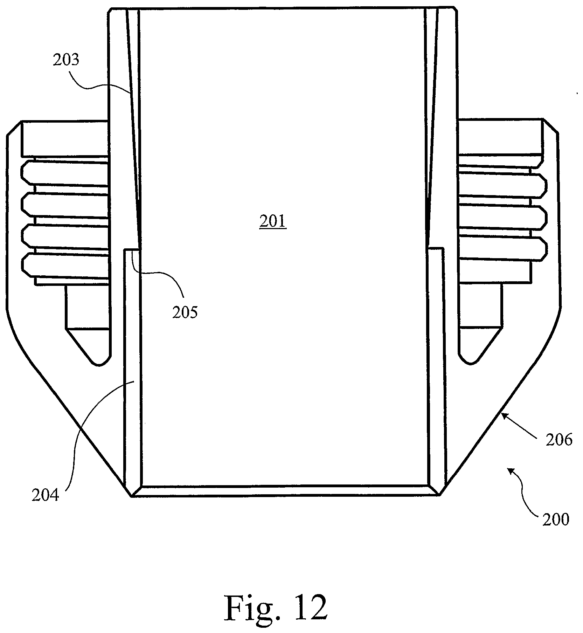

The modified front housing 200 (analogous to nozzle 11 in the prior art) is illustrated in Figure 12. The front housing 200 has a bore 201 , of sufficient diameter to allow passage therethrough of the needle 10, needle cover 17 and the front and intermediate portions 105, 106 of the syringe holder (but not the flange 104).

The interior surface of the bore 201 is provided with two (or more) equispaced longitudinal slots 202, each having a rear section 203 with a tapered surface providing a varying depth and a forward section 204 of substantially constant depth. The boundary between the forward and rear sections of each slot 202 is defined by a step 205.

The slots 202 are positioned so that they can be aligned with the tags 110 at the front portion of the syringe holder.

The slots 202 enable the syringe holder 100 to be assembled into the front housing 200 at a specific axial position (relative to the front housing) so that the flexible fingers 108 stand clear of the front housing instead of being surrounded therein.

The slots 202 also provide radial location for the syringe holder 100 as it is inserted therein.

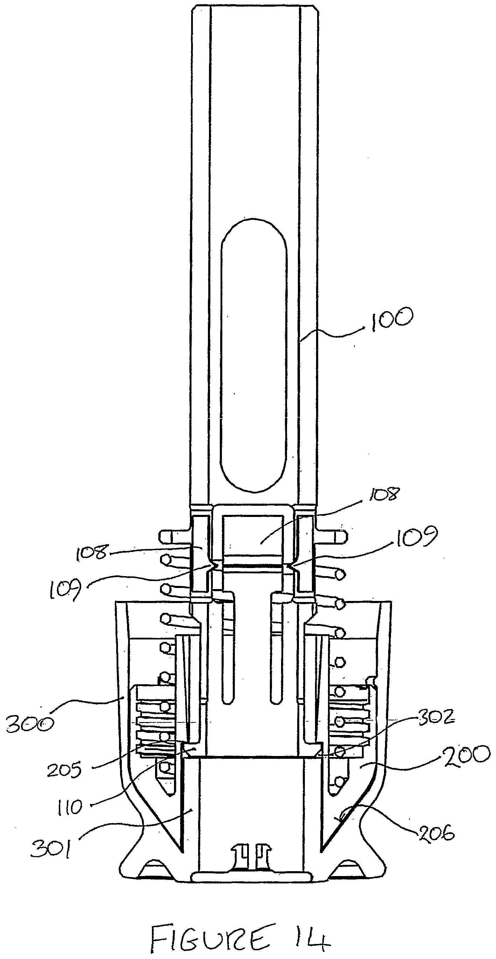

Figure 14 shows a modified end cap 300 (analogous to end cap 15 in the prior art) designed for use with the modified front housing 200 and how the syringe holder 100 is inserted into the front housing 200. The front housing 200 is inserted into the end cap 300 so that its leading surface 206 abuts the interior of the end cap, so that no further forward movement of the front housing within the end cap is possible.

The close abutment of the end cap 300, front housing 200 and syringe holder 100 means that, if present, the needle cover 17 is retained securely in position such that the risk of the needle cover accidentally becoming loose or detached is minimised, thereby minimizing possible loss of integrity of the seal between the rubber needle sheath and the needle, which would otherwise compromise the sterility of the medicament contained within the syringe. The carefully engineered interaction between the components avoids the risk that during assembly, the syringe holder 100 might undesirably "snap" in between front end of the syringe barrel and the rear of the needle cover in such a way as to compromise the seal between the end of the needle and the needle sheath in which it is staked.

The end cap has an upstanding annulus 301 which protrudes into and is a close fit in the bore 201 of the front housing 200. The upstanding annulus 301 has two equispaced protrusions on the exterior surface thereof which locate in longitudinal slots 202 when the end cap and front housing are assembled together. Once assembled together, the upstanding annulus of the end cap and the step 205 together define a space 302 into which tags 110 on the syringe holder 100 can locate.

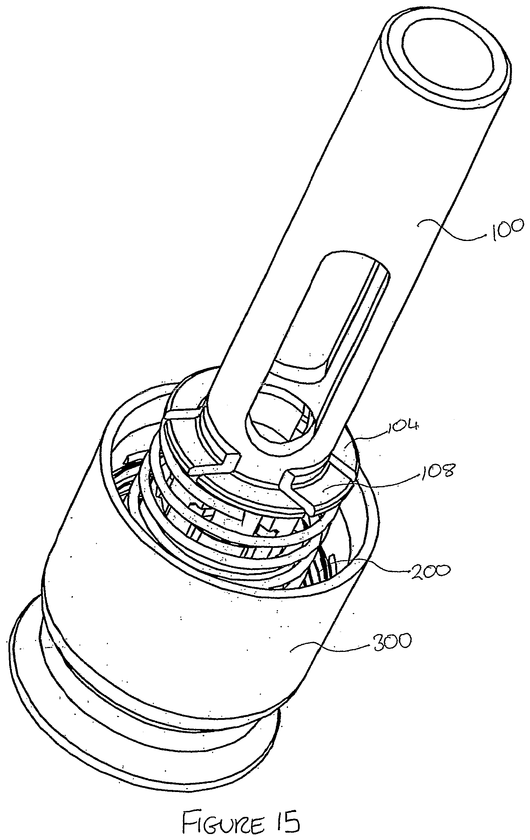

In order to insert the syringe holder 100 into the front housing 200, the tags 110 are aligned with and pushed into the slots 202 until the tags 110 "click" over the step 205 and locate in the space 302. This is the position illustrated in Figures 14 and 15. Now the syringe holder 100 is suitably axially located such that the gripping means 109 and flexible fingers 108 are not constrained within the front housing 200 and end cap 300.

The front housing, end cap and syringe holder are supplied in this ready-assembled condition, together with the ready-assembled rear part of the injection device, for final assembly with a pre-filled syringe.

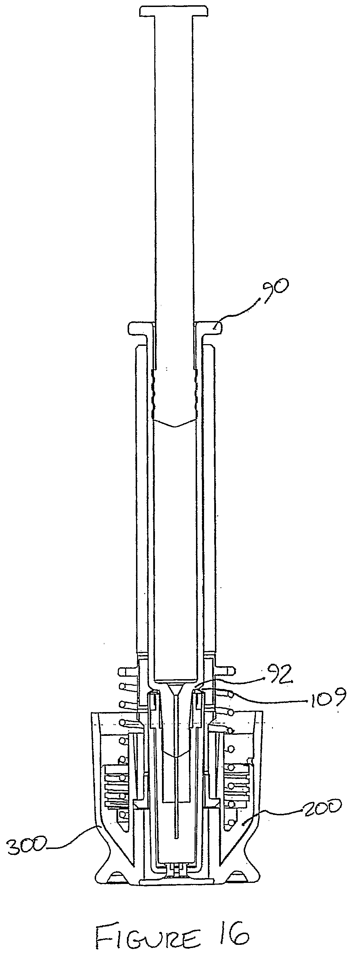

It is therefore a straightforward two-stage procedure to finally assemble the syringe into the device, namely:
1. inserting the syringe into the ready-assembled front housing, end cap and syringe holder until the gripping means 109 locate at the front shoulder 92 of the barrel (as shown in Figure 16);
2. assembling the front part to the ready-assembled rear part of the injection device (not illustrated).

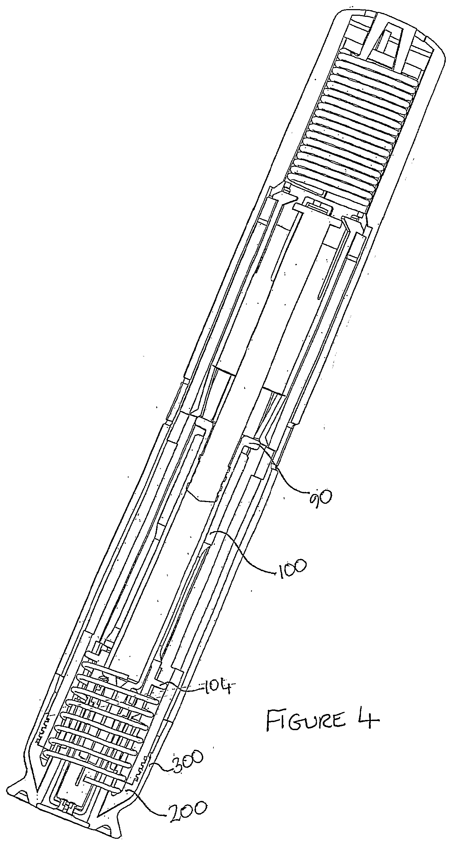

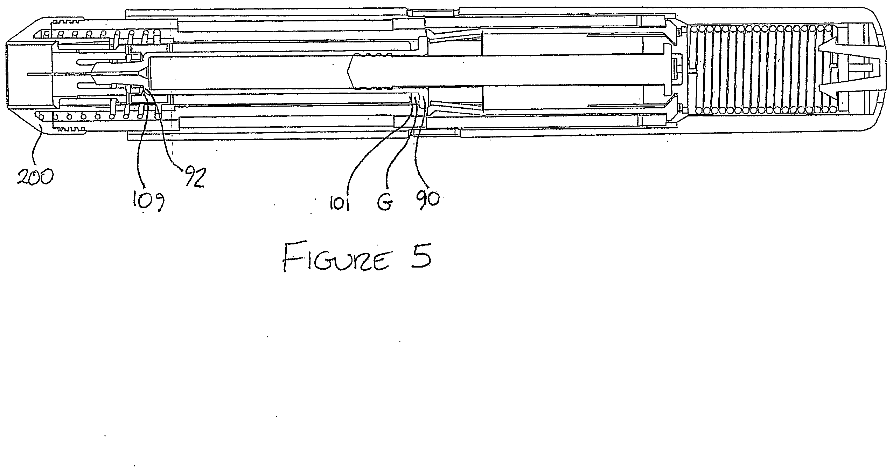

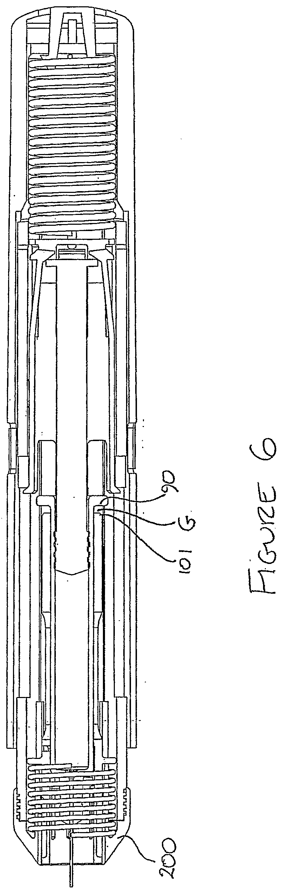

Figures 4-6 show the fully assembled injection device including syringe holder 100. With reference to Figure 5, it can be seen that (unlike in the prior art device) the flange 90 of the barrel does not contact the barrel seat 101 of the syringe holder 100, there being a gap G therebetween. This is a result of the relative axial positions of the syringe holder and syringe being determined at the front end, by gripping means 109 and front shoulder 92 and means that undesirable tension is not applied to the glass barrel during delivery of the medicament.

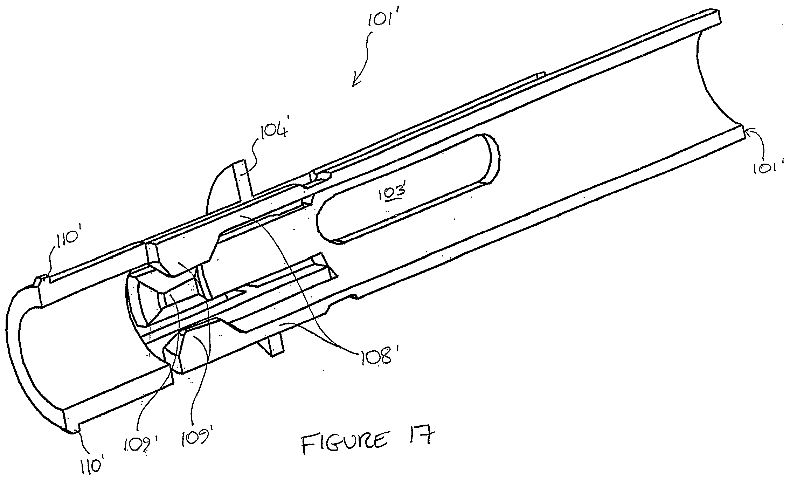

An alternative embodiment of the syringe holder is illustrated in Figures 17-20. Where possible, the same reference numerals as were used in relation to Figures 8-9 are used to identify like components of the alternative embodiment. Note that Figures 17-20 show the front end of the device at the left side of the Figures, whereas Figures 8-9 show the front end of the device at the right side of the Figures.

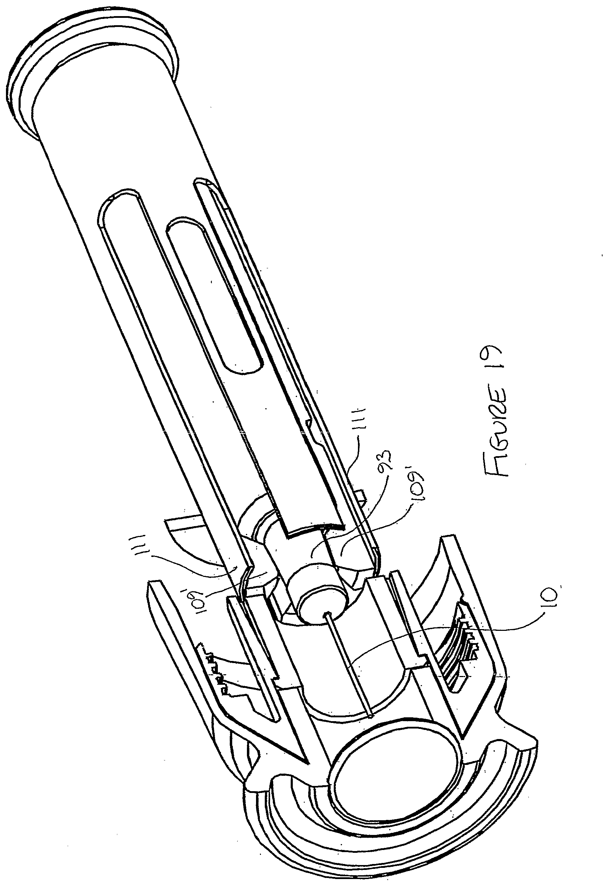

In the Figure 17 embodiment of the syringe holder 100', the radially-flexible fingers 108' have their free ends extending in the forward direction (compare with the radially-flexible fingers 108 in Figure 8 which have their free ends extending in the rearward direction). The gripping means comprise inwardly-directed enlarged heads 109' at the end of the flexible fingers 108. As is best seen in Figure 19, the enlarged heads 109' are capable of gripping the cone 93 at the front of the syringe barrel. The cone 93 is the region where the needle 10 is attached to the syringe barrel.

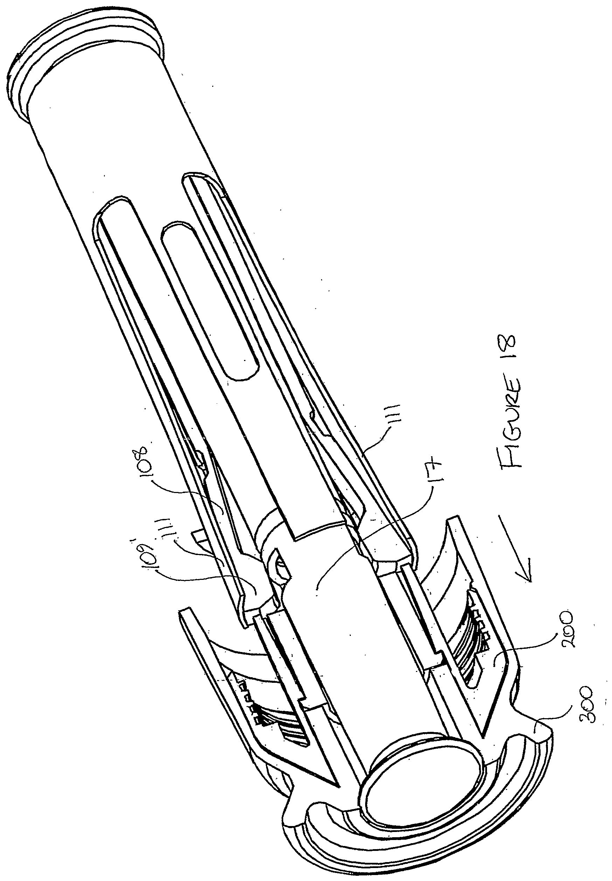

As shown in Figure 18, when a syringe is assembled with the syringe holder 100', the needle cover 17 (which is of comparable diameter to the syringe barrel) causes the flexible fingers 108' to flex radially-outwardly so that the enlarged heads 109' rest on the exterior of the needle cover 17.

When the end cap 300 and needle cover 17 are removed axially in the direction of the arrow in Figure 18, the enlarged heads 109' should move radially-inwardly into contact with the cone 93 of the syringe so as to grip the front end of the barrel to provide the compressive force during injection. However, if the flexible fingers 108' are made from plastic and if the device is stored in the Figure 18 configuration for many months before use (both of which are likely), it is possible that the fingers 108' will no longer automatically flex properly inwardly upon removal of the needle cover 17.

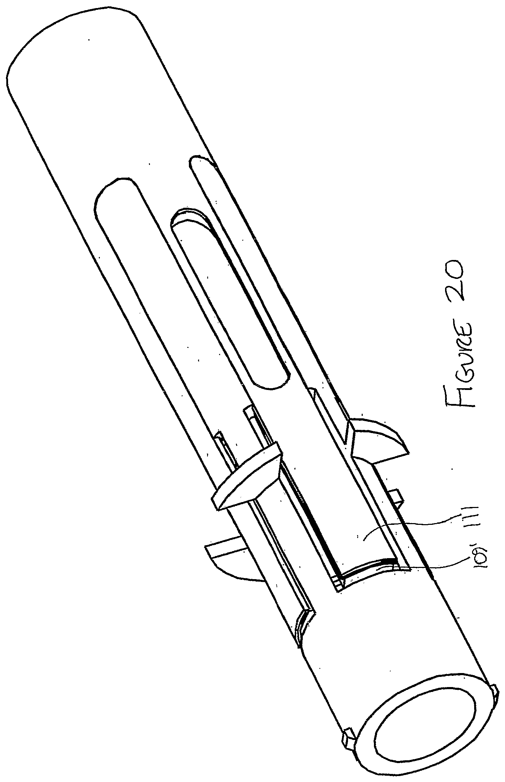

Therefore, a spring retainer 111 is provided. The spring retainer 111 is made from steel, other metal or other material which does not significantly lose its resilience over time. The spring retainer has elongate fingers which cooperate with the flexible fingers 108' so as to urge them radially-inwardly. Once the needle cover 17 has been removed, the spring retainer 111 urges the enlarged heads 109' into firm contact with the cone 93 of the syringe, even if the flexible fingers 108' are no longer capable of doing so. This position is illustrated in Figures 19 and 20.

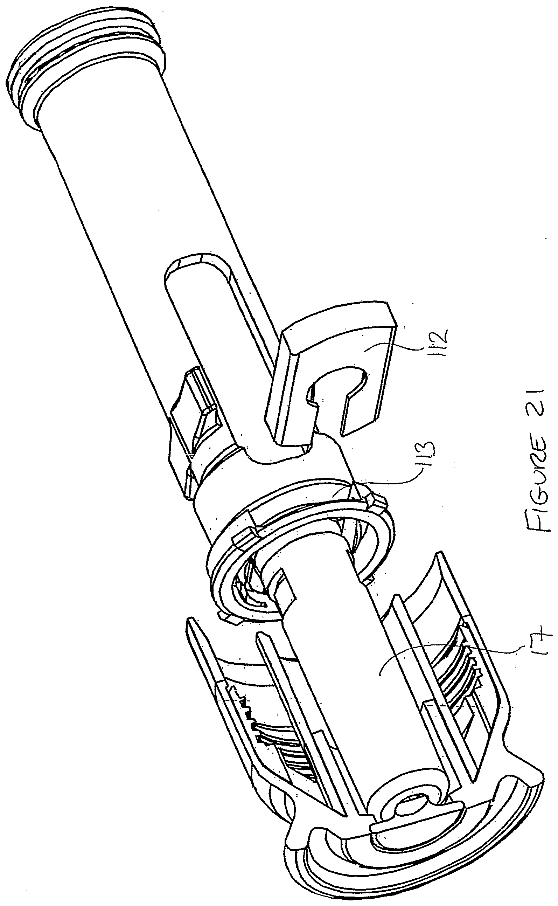

As illustrated in Figure 21 , the gripping means (109 or 109') could alternatively be provided in the form of a clip 112, inserted axially into a slot 113 in the syringe holder so as to clip onto the front of the barrel.

Other means of providing a reaction surface for the front of the barrel so as to provide a compressive force during injection can be envisaged, for example a tapered elastomeric bush which could be snapped into place once the needle cover has passed during assembly. It may be possible to grip the frontmost part of the cone, where the needle enters the cone, rather than gripping the exterior thereof. Alternatively, other means for gripping the front shoulder of the barrel, or the exterior of the cone may be envisaged.

It is observed that the viewing windows 103, 103' in the syringe holder are apertures which create space through which glass fragments could move in the event of a breakage. If a breakage occurs, it is highly desirable to contain any glass fragments so as to minimise the risk of injury. Therefore, in an alternative embodiment (not
illustrated), the apertures of the viewing windows are replaced by a solid but transparent or partially transparent material which still permits the user to view the interior of the syringe. Either the apertures 103, 103' can be "filled" with a transparent material so as to form a window in the more conventional sense of the word; alternatively, all of part of the syringe holder may be manufactured from transparent or partially transparent material.

All of the features disclosed in this specification (including any accompanying claims, abstract and drawings), and/or all of the steps of any method or process so disclosed, may be combined in any combination, except combinations where at least some of such features and/or steps are mutually exclusive.

Each feature disclosed in this specification (including any accompanying claims, abstract and drawings), may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of any foregoing embodiments but is defined by the appended claims. The invention extends to any novel one, or any novel combination, of the features disclosed in this specification (including any accompanying claims, abstract and drawings), or to any novel one, or any novel combination, of the steps of any method or process so disclosed.

## Claims

1. A unit comprising a syringe and an autoinjector comprising a housing in which can be mounted the syringe, the syringe comprising:
a barrel for holding a volume of medicament,
a needle (10) at one end of the barrel in fluid communication with the medicament,
a plunger axially-moveable in the barrel to a forwardmost position reached at the end of an injection,
the autoinjector further comprising
a needle sheath (16) which is capable of sealing the forwardmost end of the needle (10) to maintain sterility of the medicament within the barrel whereby, in use, the needle sheath (16) must be removed from the needle (10) immediately prior to actuating the autoinjector, and
a removable needle cover (17) for containing the needle sheath (16);
the autoinjector comprising:
a syringe support means (100, 100') for supporting the barrel at an axial location at or forward of the forwardmost position of the plunger, the syringe support means (100, 100') comprising gripping means that, in a normal position, form a reaction surface (109, 109') for a front shoulder (92) of the barrel, the gripping means (109, 109') being inwardly-directed protrusions on the interior of radially flexible fingers (108, 108') of the syringe support means (100, 100'), the internal diameter between the gripping means (109) being smaller than the exterior diameter of the needle cover (17); and
a front housing (200) for receiving the syringe support means (100, 100');
wherein the syringe support means (100, 100') is configured such that when the syringe is inserted axially into the rear end of the syringe support means (100, 100'), the flexible fingers (108, 108') flex radially outward from the normal position as the needle cover (17) passes and then spring back to the normal position, and in a subsequent configuration in which the syringe support means (100, 100') is received in the front housing (200), the flexible fingers (109) are prevented from flexing radially outward from the normal position due to abutment between the gripping means and the front housing (200);
whereby in use said reaction surface (109, 109') provides an axial compressive force on said barrel when a forward axial force is applied to the plunger.

2. The unit according to claim 1, wherein each of said one or more inwardly-directed protrusions are on the end of the one or more radially-flexible fingers (108, 108') and optionally comprises an inwardly-directed enlarged head on the or each radially-flexible finger (108, 108').

3. The unit according to any preceding claim, wherein said syringe support means (100, 100') further comprises one or more alignment tags at the front portion thereof, wherein optionally the front housing has a bore therethrough, the interior surface of the bore being provided with one or more longitudinal slots, positioned so that said alignment tags can locate therein, when said front housing and syringe support means are assembled together.

4. The unit according to any preceding claim, wherein said syringe support means (100, 100') is generally cylindrical and of a diameter less than the diameter of a finger flange (90) of the barrel so that the syringe support means (100, 100') is suitably sized to closely surround the barrel of the syringe, in use.

5. The unit according to any preceding claim, wherein a finger flange (90) of the barrel does not contact the syringe support means (100, 100') during delivery of the medicament.

6. The unit according to any preceding claim, comprising a syringe having:
a barrel for holding a volume of medicament,
a needle (10) at one end of the barrel in fluid communication with the medicament,
a plunger axially-moveable in the barrel to a forwardmost position,
a needle sheath (16) which is capable of sealing the forwardmost end of the needle (10) to maintain sterility of the medicament within the barrel whereby, in use, the needle sheath (16) must be removed from the needle (10) immediately prior to actuating the autoinjector, and
a removable needle cover (17) for containing the needle sheath (16).

## Patentansprüche

1. Einheit, umfassend eine Spritze und einen Autoinjektor, umfassend ein Gehäuse, in dem die Spritze montiert sein kann, die Spritze umfassend:
einen Zylinder zum Aufnehmen eines Medikamentenvolumens,
eine Nadel (10) an einem Ende des Zylinders in Fluidverbindung mit dem Medikament,
einen Kolben, der in dem Zylinder in eine vorderste Position axial bewegbar ist, die an dem Ende einer Injektion erreicht wird,
der Autoinjektor ferner umfassend
eine Nadelhülle (16), die in der Lage ist, das vorderste Ende der Nadel (10) abzudichten, um eine Sterilität des Medikaments innerhalb des Zylinders aufrechtzuerhalten, wobei in Verwendung die Nadelhülle (16) unmittelbar vor einem Betätigen des Autoinjektors von der Nadel (10) entfernt werden muss, und
eine entfernbare Nadelabdeckung (17) zum Enthalten der Nadelhülle (16);
der Autoinjektor umfassend:
ein Spritzenstützmittel (100, 100') zum Stützen des Zylinders an einer axialen Stelle an oder vor der vordersten Position des Kolbens, das Spritzenstützmittel (100, 100') umfassend Greifmittel, die in einer Normalposition eine Reaktionsoberfläche (109, 109') für eine vordere Schulter (92) des Zylinders ausbilden, wobei die Greifmittel (109, 109') nach innen gerichtete Vorsprünge an der Innenseite von radial flexiblen Fingern (108, 108') des Spritzenstützmittels (100, 100') sind, wobei der Innendurchmesser zwischen den Greifmitteln (109) geringer als der Außendurchmesser der Nadelabdeckung (17) ist; und
ein vorderes Gehäuse (200) zum Aufnehmen des Spritzenstützmittels (100, 100');
wobei das Spritzenstützmittel (100, 100') derart konfiguriert ist, dass, wenn die Spritze in das hintere Ende des Spritzenstützmittels (100, 100') axial eingeführt wird, sich die flexiblen Finger (108, 108') aus der Normalposition radial nach außen biegen, während die Nadelabdeckung (17) passiert, und dann in die Normalposition zurückspringen, und in einer nachfolgenden Konfiguration, in der das Spritzenstützmittel (100, 100') in dem vorderen Gehäuse (200) aufgenommen ist, die flexiblen Finger (109) aufgrund eines Anliegens zwischen dem Greifmittel und dem vorderen Gehäuse (200) daran gehindert werden, sich aus der Normalposition radial nach außen zu biegen;
wobei in Verwendung die Reaktionsoberfläche (109, 109') eine axiale Druckkraft auf den Zylinder bereitstellt, wenn eine nach vorne gerichtete axiale Kraft auf den Kolben ausgeübt wird.

2. Einheit nach Anspruch 1, wobei jeder des einen oder der mehreren nach innen gerichteten Vorsprünge an dem Ende des einen oder der mehreren radial flexiblen Finger (108, 108') ist und optional einen nach innen gerichteten vergrößerten Kopf an dem oder jedem radial flexiblen Finger (108, 108') umfasst.

3. Einheit nach einem der vorstehenden Ansprüche, wobei das Spritzenstützmittel (100, 100') ferner einen oder mehrere Ausrichtungsansätze an dem vorderen Abschnitt davon umfasst, wobei optional das vordere Gehäuse eine Bohrung dahindurch aufweist, wobei die innere Oberfläche der Bohrung mit einem oder mehreren Längsschlitzen versehen ist, die positioniert sind, sodass sich die Ausrichtungsansätze darin anordnen können, wenn das vordere Gehäuse und das Spritzenstützmittel zusammengebaut sind.

4. Einheit nach einem der vorstehenden Ansprüche, wobei das Spritzenstützmittel (100, 100') im Allgemeinen zylindrisch ist und einen Durchmesser aufweist, der geringer als der Durchmesser eines Fingerflansches (90) des Zylinders ist, sodass das Spritzenstützmittel (100, 100') passend bemessen ist, um den Zylinder der Spritze in Verwendung eng zu umschließen.

5. Einheit nach einem der vorstehenden Ansprüche, wobei ein Fingerflansch (90) des Zylinders das Spritzenstützmittel (100, 100') während einer Abgabe des Medikaments nicht berührt.

6. Einheit nach einem der vorstehenden Ansprüche, umfassend eine Spritze, die aufweist:
einen Zylinder zum Aufnehmen eines Medikamentenvolumens,
eine Nadel (10) an einem Ende des Zylinders in Fluidverbindung mit dem Medikament,
einen Kolben, der in dem Zylinder in eine vorderste Position axial bewegbar ist,
eine Nadelhülle (16), die in der Lage ist, das vorderste Ende der Nadel (10) abzudichten, um eine Sterilität des Medikaments innerhalb des Zylinders aufrechtzuerhalten, wobei in Verwendung die Nadelhülle (16) unmittelbar vor einem Betätigen des Autoinjektors von der Nadel (10) entfernt werden muss, und
eine entfernbare Nadelabdeckung (17) zum Enthalten der Nadelhülle (16).

## Revendications

1. Unité comprenant une seringue et un auto-injecteur comprenant un logement dans lequel peut être montée la seringue, la seringue comprenant :
un corps permettant de contenir un volume de médicament,
une aiguille (10) au niveau d'une extrémité du corps en communication fluidique avec le médicament,
un piston pouvant être déplacé axialement dans le corps jusqu'à une position la plus en avant atteinte à la fin d'une injection,
l'auto-injecteur comprenant en outre
une gaine d'aiguille (16) qui est capable de sceller l'extrémité la plus en avant de l'aiguille (10) pour maintenir la stérilité du médicament à l'intérieur du corps, moyennant quoi, lors de l'utilisation, la gaine d'aiguille (16) doit être retirée de l'aiguille (10) immédiatement avant l'actionnement de l'auto-injecteur, et
un capuchon d'aiguille (17) amovible permettant de contenir la gaine d'aiguille (16) ;
l'auto-injecteur comprenant :
un moyen de support de seringue (100, 100') permettant de supporter le corps au niveau d'un emplacement axial au niveau ou en avant de la position la plus en avant du piston, le moyen de support de seringue (100, 100') comprenant des moyens de préhension qui, dans une position normale, forment une surface de réaction (109, 109') pour un épaulement avant (92) du corps, les moyens de préhension (109, 109') étant des saillies dirigées vers l'intérieur sur l'intérieur de doigts radialement flexibles (108, 108') du moyen de support de seringue (100, 100'), le diamètre interne entre les moyens de préhension (109) étant inférieur au diamètre extérieur du capuchon d'aiguille (17) ; et
un logement avant (200) permettant de recevoir les moyens de support de seringue (100, 100') ;
dans lequel le moyen de support de seringue (100, 100') est conçu de telle sorte que, lorsque la seringue est insérée axialement dans l'extrémité arrière du moyen de support de seringue (100, 100'), les doigts flexibles (108, 108') fléchissent radialement vers l'extérieur depuis la position normale à mesure que le capuchon d'aiguille (17) passe, puis reviennent à la position normale, et dans une configuration ultérieure dans laquelle le moyen de support de seringue (100, 100') est reçu dans le logement avant (200), les doigts flexibles (109) sont empêchés de fléchir radialement vers l'extérieur par rapport à la position normale en raison d'une butée entre les moyens de préhension et le logement avant (200) ;
moyennant quoi, lors de l'utilisation, ladite surface de réaction (109, 109') fournit une force de compression axiale sur ledit corps lorsqu'une force axiale vers l'avant est appliquée au piston.

2. Unité selon la revendication 1, dans laquelle chacune de ladite ou desdites saillies dirigées vers l'intérieur se trouve sur l'extrémité du ou des doigts radialement flexibles (108, 108') et comprend facultativement une tête élargie dirigée vers l'intérieur sur le ou chaque doigt radialement flexible (108, 108').

3. Unité selon l'une quelconque revendication précédente, dans laquelle ledit moyen de support de seringue (100, 100') comprend en outre une ou plusieurs étiquettes d'alignement sur la partie avant de celui-ci, dans laquelle facultativement le logement avant a un alésage à travers celui-ci, la surface intérieure de l'alésage étant pourvue d'une ou plusieurs fentes longitudinales, positionnées de sorte que lesdites étiquettes d'alignement peuvent s'y trouver, lorsque ledit logement avant et ledit moyen de support de seringue sont assemblés ensemble.

4. Unité selon l'une quelconque revendication précédente, dans laquelle ledit moyen de support de seringue (100, 100') est généralement cylindrique et d'un diamètre inférieur au diamètre d'une collerette (90) du corps de sorte que le moyen de support de seringue (100, 100') est convenablement dimensionné pour entourer étroitement le corps de la seringue, lors de l'utilisation.

5. Unité selon l'une quelconque revendication précédente, dans laquelle une collerette (90) du corps n'entre pas en contact avec le moyen de support de seringue (100, 100') pendant l'administration du médicament.

6. Unité selon l'une quelconque revendication précédente, comprenant une seringue ayant :
un corps permettant de contenir un volume de médicament,
une aiguille (10) au niveau d'une extrémité du corps en communication fluidique avec le médicament,
un piston pouvant être déplacé axialement dans le corps jusqu'à une position la plus en avant,
une gaine d'aiguille (16) qui est capable de sceller l'extrémité la plus en avant de l'aiguille (10) pour maintenir la stérilité du médicament à l'intérieur du corps, moyennant quoi, lors de l'utilisation, la gaine d'aiguille (16) doit être retirée de l'aiguille (10) immédiatement avant l'actionnement de l'auto-injecteur, et
un capuchon d'aiguille (17) amovible permettant de contenir la gaine d'aiguille (16).
